# EUROPÄISCHE PATENTSCHRIFT

(11) **EP 2 018 828 B1**
(45) Veröffentlichungstag und Bekanntmachung des Hinweises auf die Patenterteilung: **06.05.2015**
(21) Anmeldenummer: 08013272.3
(22) Anmeldetag: 23.07.2008
(51) Int. Cl.: A61B 17/80, A61B 17/86, A61B 17/32, A61B 17/00

(54) **Medizinisches Synthesesystem**
Medical synthesis system
Système de synthèse médical

(30) Priorität: 23.07.2007 DE 102007034169
(43) Veröffentlichungstag der Anmeldung: 28.01.2009
(73) Patentinhaber: Karl Leibinger Medizintechnik GmbH & Co. KG, 78570 Mühlheim/Donau (DE)
(72) Erfinder: Gabele, Lorenz, 88605 Sauldorf (DE); Reinauer, Frank, 78576 Emmingen-Liptingen (DE); Mayer, Jörg, Dr., CH-5702 Niederlenz (CH)
(74) Vertreter: Laufhütte, Dieter

(56) Entgegenhaltungen:
- EP-B1- 1 363 543
- US-A1- 2001 002 439
- US-A1- 2005 216 008

## Beschreibung

Die Erfindung betrifft ein medizinisches Synthesesystem gemäß dem Oberbegriff von Patentanspruch 1.

Mehrteilige medizinische Synthesesysteme sind aus dem Stand der Technik bekannt. Diese vorbekannten Systeme bestehen üblicherweise aus tragenden bzw. fixierenden Elementen wie Schrauben oder Nägeln sowie Syntheseplatten. Derartige Systeme können beispielsweise dafür eingesetzt werden, Knochenfragmente miteinander zu verbinden. Dazu wird bislang die Syntheseplatte auf die zu verbindenden Knochenfragmente aufgelegt und mittels Schrauben oder Nägeln befestigt. Dies weist aber den Nachteil auf, dass durch die Befestigung die Syntheseplatte in ihrer Position verändert werden kann. Selbst größte Erfahrung der anbringenden Person kann in bestimmten Fällen derartige Ungenauigkeiten nicht vermeiden, da zumeist der räumliche Zugang sowie die Sicht auf das zu befestigende Synthesesystem stark eingeschränkt ist.

Ein medizinisches Synthesesystem mit den Merkmalen des Oberbegriffs ist aus der US 2001/002439 A1 bekannt.

Es ist daher die Aufgabe der vorliegenden Erfindung, ein medizinisches Synthesesystem dahingehend weiterzubilden, dass die Verbindung der Komponenten des medizinischen Synthesesystems mit größtmöglicher Genauigkeit auch unter beengten räumlichen Gegebenheiten sowie unter schlechten Sichtverhältnissen durchgeführt werden kann.

Diese Aufgabe wird durch ein medizinisches Synthesesystem mit den Merkmalen des Anspruchs 1 gelöst. Vorteilhafte Weiterbildungen der Erfindung sind Gegenstand der abhängigen Ansprüche.
Danach ist es vorgesehen, dass das medizinische Synthesesystem wenigstens ein tragendes Element und wenigstens ein Syntheseelement umfasst, wobei das tragende Element für die Befestigung in einer organischen Struktur vorgesehen ist. Dabei ist das Syntheseelement für die Befestigung an dem bereits befestigten tragenden Element vorgesehen. Das Syntheseelement und das tragende Element bilden während der Anwendung eine nicht lösbare zusammenhängende Einheit.

Der Begriff "während der Anwendung" bezeichnet dabei den Zeitraum ab Montage des medizinischen Synthesesystem bis zur Entfernung des selben oder bis zur Resorption wenigstens von Teilen des medizinischen Synthesesystems. Ferner wird für die Definition des Begriffes davon ausgegangen, dass während der Anwendung keine äußeren Einflüsse zum Versagen des Systems führen.

Ein medizinisches Synthesesystem mit den Merkmalen des Anspruchs 1 weist somit den Vorteil auf, dass zunächst z.B. mehrere tragende Elemente wie Schrauben sehr genau positioniert und befestigt werden können. Erfindungsgemäß ist dann eine Syntheseplatte auf den bereits befestigten Schrauben auf den Schraubenköpfen angebracht. Eine derartige Verbindung kann einfach, schnell und sicher realisiert werden. Ein derartiges System erlaubt einen Zusammenbau mit größtmöglicher Positionierungsgenauigkeit auf engstem Raum auch unter geringen Sichtverhältnissen. Selbstverständlich kann ein derartiges Synthesesystem zu medizinischen Aus- und Weiterbildungszwecken eingesetzt werden.

Übertragen auf den speziellen Fall der Osteosynthese wird somit eine minimalinvasive und hochgenaue Positionierung möglich. Diese geht zudem mit einer signifikanten Zeitersparnis einher. Eine Zeitersparnis im Gesundheitswesen geht zudem mit einer großen Kostenersparnis einher, da einerseits entsprechende Räumlichkeiten wie Operationssäle besser ausgelastet werden können, andererseits ist an einem Osteosynthesevorgang auch immer ein Team von mehreren Personen beteiligt.

Es kann darüber hinaus vorgesehen sein, dass das Syntheseelement mittels einer durch Energieeintrag und/oder durch Wärme und/oder Ultraschallschweißen und/oder Reibung gebildeten Verbindung an dem tragenden Element befestigt ist. dadurch ergibt sich der Vorteil, dass eine Verbindung schnell und zuverlässig hergestellt werden kann. Diese Verbindung kann zugleich auch sehr genau an einer vordefinierten Position hergestellt werden. Im medizinischen Bereich sind für die Erstellung derartiger Verbindungen Schweißverfahren bekannt, die sich im entsprechenden Zusammenhang bereits bewährt haben.

Das Syntheseelement kann an dem tragenden Element befestigt sein, wobei die Verbindung der beiden Elemente mit Einsatz eines Hilfsstoffes hergestellt wird. Ein resorbierbarer oder ein nicht resorbierbarer biokompatibler Werkstoff kann in diesem Fall zwischen das Syntheseelement sowie das tragende Element eingebracht werden. Durch Energieeintrag und/oder durch Wärme und/oder Ultraschallschweißen und/oder Reibung werden die Komponenten verbunden. Dadurch ergibt sich der Vorteil, Komponenten eines Synthesesystems miteinander verschweißen zu können, die aus unterschiedlichen und zumindest einem nicht verschweißbaren Werkstoff bestehen. Durch den Hilfsstoff wird dann z.B. eine Schweißverbindung möglich.

Das tragende Element kann ferner in der organischen Struktur durch Einschrauben oder Eintreiben oder Einschweißen befestigt sein. Dies ermöglicht eine hochgenaue Positionierung des tragenden Elements. Ein Einschweißverfahren kann beispielsweise das sogenannte "sonic-weld-Verfahren" sein. Es kann dabei vorgesehen sein, dass das tragende Element eine Schraube, ein Pin oder ein Nagel ist.

Besonders vorteilhaft ist es, wenn das tragende Element aus einem resorbierbaren Werkstoff besteht. Eine Resorption des tragenden Elements kann eine Entfernung nach der Anwendung überflüssig machen. Beispielhaft für die Osteosynthese sei angeführt, dass bei Verwendung des erfindungsgemäßen Systems eine verbesserte und schnellere Heilung aufgrund reduzierten Operationstraumas möglich ist und im Falle des Einsatzes von resorbierbaren Materialien auf eine Entfernung verzichtet werden kann.

Das Syntheseelement kann als Platte oder als Folie oder als Membran oder als Netz ausgeführt sein. Eine Platte weist beispielsweise den Vorteil auf, dass zu verbindende Fragmente sicher während der Anwendung miteinander verbunden werden können.

Weiterhin ist denkbar, dass das Syntheseelement aus einem thermoplastischen Werkstoff besteht. Thermoplastische Werkstoffe weisen den Vorteil auf, dass mit ihnen in Folge von Wärmeeintrag stoffschlüssige Verbindungen realisiert werden können.

Von besonderem Vorteil ist es, wenn das Syntheseelement aus einem durchsichtigen Werkstoff besteht. Dadurch wird es der das Synthesesystem verbindenden Person möglich, zunächst die Syntheseelemente relativ zu den tragenden Elementen zu positionieren und nach der exakten Positionierung die Schweißverbindung z.B. mittels Ultraschallschweißen zu realisieren.

Das Syntheseelement kann weiterhin aus einem resorbierbaren Werkstoff bestehen. Insbesondere in Kombination mit resorbierbaren tragenden Elementen ergibt sich der Vorteil, dass auf eine gesonderte Entfernung verzichtet werden kann. Darüber hinaus ist vorteilhaft, dass resorbierbare Werkstoffe für medizinische Anwendungen eine hohe Biokompatibilität aufweisen und zumindest einige Werkstoffe thermoplastisch sind. Mehrere vorgenannte Vorteile werden somit vereint. Insbesondere können die Komponenten neben der exakten Positionierung direkt miteinander stoffschlüssig verbunden werden.

Für den Einsatz als Osteosynthesesystem ergibt sich der Vorteil, dass neben einer exakten Fixierung von Knochenfragmenten oder dem Ersatz fehlender Fragmente oder organischer Strukturen durch den resorbierbaren Werkstoff die Zeit der notwendigen Anwendung verringert werden kann.

Darüber hinaus wird ein Verfahren zum Verbinden eine mehrteiligen medizinischen Synthesesystems beschrieben, bestehend aus wenigstens einem tragenden Element, wobei das tragende Element für die Befestigung in einer organischen Struktur vorgesehen ist, und wenigstens einem Syntheseelement das Syntheseelement an dem bereits befestigten tragenden Element zusammenhängend und nicht lösbar für die Anwendung verbunden wird.

Die Vorteile des Verfahrens ergeben sich beispielweise nach der Positionierung der tragenden Elemente. Es kann dann mit wenig Aufwand z.B. eine Syntheseplatte auf den bereits befestigten Schrauben auf dem Schraubenköpfen angebracht werden. Ein derartiges System erlaubt einen schnellen und sicheren Zusammenbau mit größtmöglicher Positionierungsgenauigkeit auf engstem Raum auch unter erschwerten Sichtverhältnissen.

Das Syntheseelement kann mittels Energieeintrag durch Wärme und/oder Ultraschallschweißen und/oder Reibung an dem tragenden Element befestigt werden. Dies ermöglicht eine schnelle und sichere stoffschlüssige Verbindung.

Es ist möglich, das Syntheseelement mittels eines resorbierbaren oder eines nicht resorbierbaren biokompatiblen Werkstoffs sowie Energieeintrag und/oder durch Wärme und/oder Ultraschallschweißen und/oder Reibung an dem tragenden Element zu befestigen. So können z.B. Werkstoffe, die nicht miteinander durch Schweißen verbunden werden können, unter Zuhilfenahme eines Hilfsstoffes schnell und sicher miteinander verbunden werden.

Weitere Einzelheiten und Vorteile der Erfindung werden anhand eines in der Zeichnung dargestellten Ausführungsbeispiels näher erläutert:
Es zeigen:
   - Fig. 1:: eine Seitenansicht des erfindungsgemäßen medizinischen Synthesesystems,
   - Fig. 2:: eine Seitenansicht des erfindungsgemäßen medizinischen Synthesesystems mit einem Ultraschallschweißkopf,
   - Fig. 3:: eine Seitenansicht des Synthesesystems, wobei die Verbindung zwischen tragendem Element und Syntheselement mittels eines Hilfsstoffes hergestellt wird,
   - Fig. 4:: eine Seitenansicht eines tragenden Elements mit einer Befestigungsflä- che.

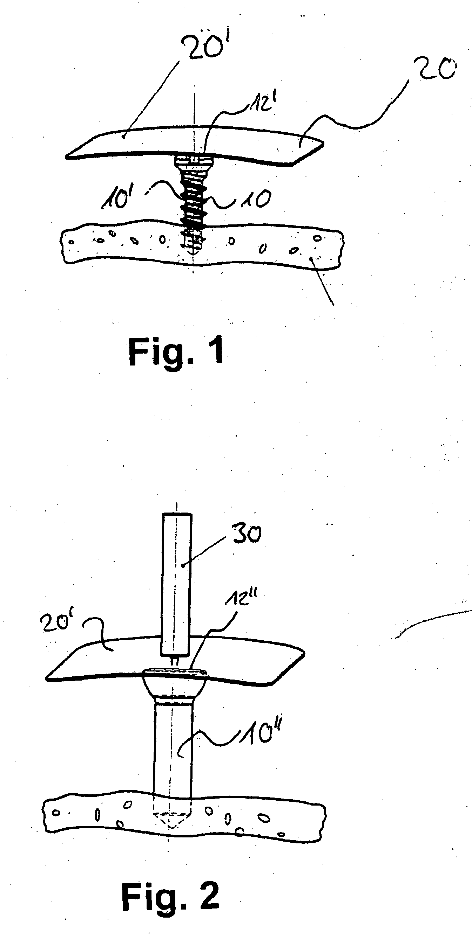

Die Figur 1 zeigt das erfindungsgemäße medizinische Synthesesystem in einer Ausführungsform mit einer Schraube 10' als tragendem Element 10. Die Schraube 10' aus resorbierbarem, thermoplastischem Kunststoff ist dabei bereits befestigt. Auf dem Schraubenkopf 12' wird das als Syntheseplatte 20' ausgeführte Syntheseelement 20 aufgeschweißt. Die Syntheseplatte 20' ist dabei aus einem durchsichtigen, resorbierbaren und thermoplastischen Kunststoff ausgeführt. Dies ermöglicht eine exakte, schnelle und sichere Positionierung der Syntheseplatte 20' relativ zur Schraube 10'.

Die Figur 2 zeigt das erfindungsgemäße medizinische Synthesesystem in einer Ausführungsform mit einem Pin 10" als tragendem Element. Dieser Pin 10" ist dabei ebenfalls aus resorbierbarem, thermoplastischem Kunststoff. Mittels eines Ultraschallschweißkopfes 30 wird die Syntheseplatte 20' auf den Kopf 12" des Pins 10" aufgeschweißt. Die dabei entstehenden Temperaturen sind dabei für die umgebenden Strukturen unbedenklich. Die Syntheseplatte 20' ist dabei aus einem durchsichtigen, resorbierbaren und thermoplastischen Kunststoff ausgeführt, was eine exakte Positionierung von Schweißkopf 30, Syntheseplatte 20' und Pin 10" ermöglicht.

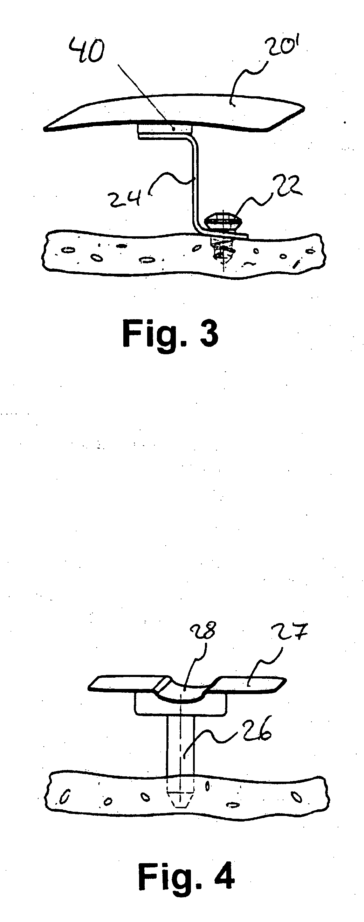

In Figur 3 ist die Ausführungsform gezeigt, bei der für die Verbindung der Komponenten des medizinischen Synthesesystems ein gesonderter Hilfsstoff 40 aus thermoplastischem, biokompatiblem Material benötigt wird. Das tragende Element besteht dabei aus einer Schraube 22 sowie einem Haltebügel 24 aus einer Metalllegierung, wobei der Haltebügel 24 ohne Hilfsstoff 40 mit der Syntheseplatte nicht sicher verschweißt werden kann. Die Syntheseplatte 20' besteht in diesem Ausführungsbeispiel aus einem biokompatiblen Thermoplast.

In Figur 4 ist eine Seitenansicht eines tragenden Elements in der Ausführung als Pin 26 mit einer Befestigungsfläche 27 gezeigt. Die Befestigungsfläche 27 erlaubt dabei die sichere Auflage eines Syntheseelements, wobei zusätzliche eine Vertiefung 28 in der Befestigungsfläche 27 für die Aufnahme von Hilfsstoffen oder den plastisch verformten Materials vorgesehen ist.

## Patentansprüche

1. Medizinisches Synthesesystem mit wenigstens einem tragenden Element (10) und wenigstens einem Syntheseelement (20), wobei das tragende Element (10) für die Befestigung in einer organischen Struktur vorgesehen ist, wobei das Syntheseelement (20) für die Befestigung an dem bereits befestigten tragenden Element (10) vorgesehen ist und dass das Syntheseelement (20) und das tragende Element (10) während der Anwendung eine nicht lösbare zusammenhängende Einheit bilden,
**dadurch gekennzeichnet,**
**dass** das tragende Element (10) eine Schraube (10') mit einem Schraubenkopft (12') ist und wobei das Syntheseelement (20) am Schraubenkopf (12') aufschweißbar ist oder wobei das tragende Element (10) ein Pin (10", 26) mit einem Kopf (12") oder einer Befestigungsfläche (27) ist und wobei das Syntheseelement (20) am Kopf (12") des Pins (10") oder an der Befestigungsfläche (27) des Pins (26) mit einer Vertiefung (28) für die Aufnahme von Hilfsstoffen oder plastisch verformten Materials aufschweißbar ist oder wobei das tragende Element (10) aus einer Schraube (22) und einem Haltebügel (24) besteht und wobei das Syntheseelement (20) am Haltebügel (24) mit einem Hilfsstoff (40) aufschweißbar ist.

2. Medizinisches Synthesesystem nach Anspruch 1, **dadurch gekennzeichnet, dass** das Syntheseelement (20) mittels einer durch Energieeintrag und/oder durch Wärme und/oder Ultraschallschweißen und/oder Reibung gebildeten Verbindung an dem tragenden Element (10) befestigtbar ist.

3. Medizinisches Synthesesystem nach Anspruch 1, **dadurch gekennzeichnet, dass** das Syntheseelement (20) mittels einer durch einen resorbierbaren oder einen nicht resorbierbaren biokompatiblen Werkstoff sowie Energieeintrag und/oder durch Wärme und/oder Ultraschallschweißen und/oder Reibung gebildeten Verbindung an dem tragenden Element (10) befestigbar ist.

4. Medizinisches Synthesesystem nach Anspruch 1 oder 2, **dadurch gekennzeichnet, dass** das tragende Element (10) in der organische Struktur durch Einschrauben oder Eintreiben oder Einschweißen befestigbar ist.

5. Medizinisches Synthesesystem nach einem der vorhergehenden Ansprüche, **dadurch gekennzeichnet, dass** das tragende Element (10) aus einem biokompatiblen Werkstoff besteht.

6. Medizinisches Synthesesystem nach einem der vorhergehenden Ansprüche, **dadurch gekennzeichnet, dass** das tragende Element (10) aus einem resorbierbaren Werkstoff besteht.

7. Medizinisches Synthesesystem nach einem der vorhergehenden Ansprüche, **dadurch gekennzeichnet, dass** das Syntheseelement (20) als Platte oder als Folie oder als Membran oder als Netz ausgeführt ist.

8. Medizinisches Synthesesystem nach einem der vorhergehenden Ansprüche, **dadurch gekennzeichnet, dass** das Syntheseelement (20) aus einem thermoplastischen Werkstoff besteht.

9. Medizinisches Synthesesystem nach einem der vorhergehenden Ansprüche, **dadurch gekennzeichnet, dass** das Syntheseelement (20) aus einem durchsichtigen Werkstoff besteht.

10. Medizinisches Synthesesystem nach einem der vorhergehenden Ansprüche, **dadurch gekennzeichnet, dass** das Syntheseelement (20) aus einem resorbierbaren Werkstoff besteht.

## Claims

1. A medical synthesis system having at least one supporting element (10) and at least one synthesis element (20), wherein the supporting element (10) is provided for fastening in an organic structure, wherein the synthesis element (20) is provided for the fastening to the already fastened supporting element (10); and in that the synthesis element (20) and the supporting element (10) form a non-releasable coherent unit during the application,
**characterized in that**
the supporting element (10) is a screw (10') having a screw head (12') and wherein the synthesis element (20) can be welded onto the screw head (12') or wherein the supporting element (10) is a pin (10", 26) having a head (12") or a mounting surface (27) and wherein the synthesis element (20) can be welded onto the head (12") of the pin (10") or onto the mounting surface (27) of the pin (26) having a recess (28) for the reception of auxiliary materials or plastically deformed materials or wherein the supporting element (10) consists of a screw (22) and a retaining bracket (24) and wherein the synthesis element (20) can be welded onto the retaining bracket (24) using an auxiliary material (40).

2. A medical synthesis system in accordance with claim 1, **characterized in that** the synthesis element (20) can be fastened to the supporting element (10) by means of a connection formed by energy input and/or by heat and/or by ultrasonic welding and/or by friction.

3. A medical synthesis system in accordance with claim 1, **characterized in that** the synthesis element (20) can be fastened to the supporting element (10) by means of a connection formed by a resorbable or a non-resorbable biocompatible material as well as by energy input and/or by heat and/or by ultrasonic welding and/or by friction.

4. A medical synthesis system in accordance with either of claims 1 or 2, **characterized in that** the supporting element (10) can be fastened in the organic structure by screwing or driving or welding.

5. A medical synthesis system in accordance with one of the preceding claims, **characterized in that** the supporting element (10) comprises a biocompatible material.

6. A medical synthesis system in accordance with one of the preceding claims, **characterized in that** the supporting element (10) comprises a resorbable material.

7. A medical synthesis system in accordance with one of the preceding claims, **characterized in that** the synthesis element (20) is made as a plate or as a film or as a membrane or as a mesh.

8. A medical synthesis system in accordance with one of the preceding claims, **characterized in that** the synthesis element (20) comprises a thermoplastic material.

9. A medical synthesis system in accordance with one of the preceding claims, **characterized in that** the synthesis element (20) comprises a transparent material.

10. A medical synthesis system in accordance with one of the preceding claims, **characterized in that** the synthesis element (20) comprises a resorbable material.

## Revendications

1. Système de synthèse médical comprenant au moins un élément porteur (10) et au moins un élément de synthèse (20), l'élément porteur (10) étant destiné à la fixation dans une structure organique, l'élément de synthèse (20) étant destiné à la fixation sur l'élément porteur (10) déjà fixé et l'élément de synthèse (20) et l'élément porteur (10) formant une unité cohérente non dissociable pendant l'utilisation,
**caractérisé en ce que**
l'élément porteur (10) est une vis (10') dotée d'une tête de vis (12'), et l'élément de synthèse (20) pouvant être soudé sur la tête de vis (12') ou l'élément porteur (10) étant une broche (10", 26) dotée d'une tête (12") ou d'une surface de fixation (27) et l'élément de synthèse (20) pouvant être soudé sur la tête (12") de la broche (10") ou sur la surface de fixation (27) de la broche (26) avec un creux (28) destiné à recevoir des produits auxiliaires ou des matériaux déformés plastiquement ou l'élément porteur (10) étant constitué d'une vis (22) et d'un support de fixation (24) et l'élément de synthèse (20) pouvant être soudé sur le support de fixation (24) au moyen d'un produit auxiliaire (40).

2. Système de synthèse médical selon la revendication 1, **caractérisé en ce que** l'élément de synthèse (20) peut être fixé sur l'élément porteur (10) au moyen d'une liaison formée par apport d'énergie et/ou par la chaleur et/ou par soudage par ultrasons et/ou par friction.

3. Système de synthèse médical selon la revendication 1, **caractérisé en ce que** l'élément de synthèse (20) peut être fixé sur l'élément porteur (10) au moyen d'une liaison formée par une matière biocompatible résorbable ou non résorbable ainsi que par apport d'énergie et/ou par la chaleur et/ou par soudage par ultrasons et/ou par friction.

4. Système de synthèse médical selon la revendication 1 ou 2, **caractérisé en ce que** l'élément porteur (10) peut être fixé dans la structure organique par vissage, enfoncement ou soudage.

5. Système de synthèse médical selon l'une des revendications précédentes, **caractérisé en ce que** l'élément porteur (10) est constitué d'une matière biocompatible.

6. Système de synthèse médical selon l'une des revendications précédentes, **caractérisé en ce que** l'élément porteur (10) est constitué d'une matière résorbable.

7. Système de synthèse médical selon l'une des revendications précédentes, **caractérisé en ce que** l'élément de synthèse (20) est réalisé sous la forme d'une plaque, d'un film, d'une membrane ou d'un tissu.

8. Système de synthèse médical selon l'une des revendications précédentes, **caractérisé en ce que** l'élément de synthèse (20) est constitué d'une matière thermoplastique.

9. Système de synthèse médical selon l'une des revendications précédentes, **caractérisé en ce que** l'élément de synthèse (20) est constitué d'une matière transparente.

10. Système de synthèse médical selon l'une des revendications précédentes, **caractérisé en ce que** l'élément de synthèse (20) est constitué d'une matière résorbable.
